# EUROPEAN PATENT APPLICATION

(11) **EP 4 253 524 A1**
(43) Date of publication of application: **04.10.2023**
(21) Application number: 21898169.4
(22) Date of filing: 29.11.2021
(51) Int. Cl.: C12N 1/02, C12N 1/20, C12Q 1/04

(54) **METHOD FOR COLLECTING CELLS OF MICROORGANISM IN SPECIMEN**

(30) Priority: 30.11.2020 JP 2020198420
(71) Applicant: Kabushiki Kaisha Yakult Honsha, Tokyo, 105-8660 (JP)
(72) Inventor: KATO, Kosuke, Tokyo 105-8660 (JP); NAKAMURA, Madoka, Tokyo 105-8660 (JP); OKUMURA, Takekazu, Tokyo 105-8660 (JP); SHIDA, Kan, Tokyo 105-8660 (JP)
(74) Representative: Hartz, Nikolai
(86) International application number: PCT/JP2021/043705
(87) International publication number: WO 2022/114198

(57) **Abstract**

Provided is a method for simply and efficiently collecting microorganisms in fermented milk without affecting the state of cells.

A method for collecting microorganisms from a specimen containing fermented milk includes, in this order, steps of: (a) diluting the specimen with a buffer solution such that a protein concentration is 3 mg/mL or less; (b) performing centrifugation; and (c) washing and collecting the microorganisms.

## Description

### Technical Field

The present invention relates to a method for collecting microorganisms from a specimen containing fermented milk efficiently.

### Background Art

Separation and collection of cells from fermented milk are operations necessary for accurately grasping properties of starter cultures and microorganisms in a product. Fermented milk contains proteins such as casein, β-lactoglobulin, α-lactalbumin, lactoferrin, and immunoglobulin, and it is known that in acidic fermented milk with a lower pH, casein micelles are destabilized to form aggregates (curds). It is difficult to separate and collect cells of microorganisms from fermented milk containing proteins in a poorly solubilized state as described above.

On the other hand, conventionally, cells have been separated by using, for example, an alkaline solution and a chelating agent. In addition, there is also known a method for adjusting a specimen containing fermented milk to a pH of 5.4 or higher, and then adding an organic polymer-degrading enzyme to collect cells (Patent Literature 1).

However, such treatment affects the state of the cells in the fermented milk, particularly the surface layer structure, and there is a concern that properties and the structure of cells in the milk cannot be accurately measured.

### Citation List

### Patent Literature

Patent Literature 1: JP 6153461 B2

### Summary of Invention

### Technical Problem

The present invention relates to a method for simply and efficiently collecting microorganisms in fermented milk without affecting the state of cells.

### Solution to Problem

As a result of intensive studies, the present inventors have found that when a specimen containing fermented milk is diluted with a buffer solution having a specific concentration such that a protein concentration is equal to or less than a certain value, poorly solubilized proteins are dissolved, the specimen is subjected to centrifugation in this state to thereby be able to remove most of milk proteins, and cells in the specimen can be efficiently collected without applying excessive stress to microorganisms.

In other words, the present invention relates to 1) to 9) below.
1) A method for collecting microorganisms from a specimen containing fermented milk, the method including, in this order, steps of: (a) diluting the specimen with a buffer solution such that a protein concentration is 3 mg/mL or less; (b) performing centrifugation; and (c) washing and collecting the microorganisms.
2) The method according to 1), wherein the specimen is diluted such that a protein concentration is 2 mg/mL or less.
3) The method according to 1) or 2), wherein the buffer solution has a pH of 6.5 to 7.5.
4) The method according to any one of 1) to 3), wherein the buffer solution is a potassium phosphate buffer solution, a Tris-HCl buffer solution, or a HEPES buffer solution.
5) The method according to 4), wherein a concentration of the buffer solution is 25 mM to 1000 mM.
6) The method according to 4), wherein the buffer solution is a potassium phosphate buffer solution having a concentration of 25 mM to 100 mM.
7) The method according to any one of 1) to 6), wherein the step (a) is followed by a treatment selected from a mesh treatment, a heating treatment, a homogenizer treatment, a stomacher treatment, and an ultrasonic treatment.
8) The method according to any one of 1) to 7), wherein the microorganisms are viable
9) The method according to any one of 1) to 8), wherein the specimen is an acidic milk beverage.

### Advantageous Effects of Invention

According to the method of the present invention, the microorganisms in the fermented milk can be collected with ease at a low cost with a high yield while maintaining the state in the specimen. This makes it possible to accurately perform a quality evaluation of the specimen and an activity evaluation of the collected microorganisms.

### Description of Embodiments

The method of the present invention is a method for collecting microorganisms from a specimen containing fermented milk, the method including, in this order, steps of:
(a) diluting the specimen with a buffer solution such that a protein concentration is 3 mg/mL or less; (b) performing centrifugation; and (c) washing and collecting the microorganisms.

In the present invention, the "fermented milk" means a fermented product containing a milk-derived component. Examples of the fermented milk include: fermented products obtained by using a medium containing a milk component such as animal milk, e.g., cow's milk, goat's milk, sheep's milk, and horse's milk, reconstituted milk from milk powder and skim milk powder, and cream as a raw material, adding microorganisms such as lactic acid bacteria, Bifidobacteria, and yeast to the medium, and fermenting them (also including fermented products obtained by adding viable microorganisms to, for example, milk beverages); processed products thereof; diluted products thereof; and treated products thereof. The fermented milk is not limited to fermented milk defined by the Ministerial Ordinance on Milk and Milk products Concerning Compositional Standards, etc. (Ministerial Ordinance on Milk, etc.).

The "specimen containing fermented milk" preferably has a pH lower than 5.4, more preferably has a pH of 3.0 to 5.2, and particularly preferably has a pH of 3.4 to 5.2.

Examples of a form of the specimen include food and drink, cosmetics, and pharmaceuticals, and the food and drink or the cosmetics are preferable, the food and drink are more preferable, and acidic milk beverages are particularly preferable. Among the specimens, for example, the food and drink such as fermented milk, dairy products, and acidic milk beverages including lactic acid bacteria beverages as described in the Ministerial Ordinance on Milk, etc. usually contain viable cells and dead cells, and, for example, processed foods subjected to heat sterilization contain only dead cells. The method of the present invention can be used for a specimen containing either or both of viable cells and dead cells, and is particularly suitably used for a specimen containing viable cells. Here, the viable cells refer to cells having colony-forming ability. The dead cells refer to cells having no colony-forming ability, and include not only dead cells but also cells without colony-forming ability but with membrane stability and enzyme activity.

In the present invention, the "microorganisms" are not particularly limited, and examples thereof include: lactic acid bacteria, for example, Lactobacillus bacteria such as Lactobacillus casei, Lactobacillus acidophilus, Lactobacillus plantarum, Lactobacillus brevis, Lactobacillus coryniformis, Lactobacillus gasseri, Lactobacillus zeae, Lactobacillus johnsonii, Lactobacillus delbrueckii subspecies delbrueckii, and Lactobacillus delbrueckii subspecies bulgaricus, Streptococcus bacteria such as Streptococcus thermophilus, Lactococcus bacteria such as Lactococcus lactis, Lactococcus plantarum, and Lactococcus raffinolactis, Leuconostoc bacteria such as Leuconostoc mesenteroides, Leuconostoc mesenteroides subspecies cremoris, and Leuconostoc lactis, and Enterococcus bacteria such as Enterococcus faecalis and Enterococcus faecium; Bifidobacterium bacteria such as Bifidobacterium bifidum, Bifidobacterium breve, Bifidobacterium longum, Bifidobacterium animalis, Bifidobacterium adolescentis, Bifidobacterium angulatum, Bifidobacterium catenulatum, and Bifidobacterium pseudocatenulatum; and yeast, for example, Saccharomyces such as Saccharomyces cerevisiae, and Schizosaccharomyces.

Among these, the lactic acid bacteria and the Bifidobacterium bacteria are preferable from a viewpoint of the collection rate of microorganisms, and, for example, Lactobacillus bacteria, Streptococcus bacteria, and Lactococcus bacteria are preferable as the lactic acid bacteria. Note that one or more kinds of these microorganisms may be contained.

The step (a) of the present invention is a step of diluting the specimen with the buffer solution such that the protein concentration is 3 mg/mL or less.

Dilution is performed by collecting a certain amount of the specimen and adding the buffer solution thereto, and when doing so, the dilution is performed such that the protein concentration in the diluted solution is 3 mg/mL or less. At a concentration of more than 3 mg/mL, proteins may not be sufficiently removed, and there may be a problem in collecting cells.

Here, from a viewpoint of the protein removal rate and the cell collection rate, the protein concentration is preferably 2 mg/mL or less, more preferably 1.5 mg/mL or less, and more preferably 0.7 mg/mL or less.

Examples of preparation of the diluted solution include a situation of adding 10 to 40 folds, preferably 20 to 40 folds of a buffer solution to a specimen solution.

As the buffer solution used for dilution, a buffer solution exhibiting pH buffering ability around pH 7 is preferable, and specific examples thereof include a buffer solution having a pH of 6.5 to 7.5.

Examples of the buffer solution include a potassium phosphate buffer solution, a Tris-HCl buffer solution, a HEPES buffer solution, a sodium phosphate buffer solution, phosphate-buffered saline, a citrate-phosphate buffer solution, Tris-buffered saline, a bicarbonate buffer solution, an MES buffer solution, a Bis-Tris buffer solution, an ADA buffer solution, a PIPES buffer solution, an ACES buffer solution, an MOPSO buffer solution, a BES buffer solution, a TES buffer solution, a DIPSO buffer solution, a TAPSO buffer solution, a POPSO buffer solution, a HEPPSO buffer solution, and an EPPS buffer solution. Among these, the potassium phosphate buffer solution, the Tris-HCl buffer solution, and the HEPES buffer solution are preferable, and the concentration thereof is 25 mM to 1000 mM.

More specific examples thereof include a potassium phosphate buffer solution having a concentration of 25 mM to 100 mM, a Tris-HCl buffer solution having a concentration of 100 mM to 1000 mM, and a HEPES buffer solution having a concentration of 50 mM to 500 mM, and preferably a potassium phosphate buffer solution having a concentration of 25 mM to 100 mM.

In the method of the present invention, following the dilution step of (a) described above, a treatment selected from a mesh treatment, a heating treatment, a homogenizer treatment, a stomacher treatment, and an ultrasonic treatment can be performed. This increases the protein removal rate.

Here, the mesh treatment is a treatment of removing particles larger than the microorganism using a filter having mesh holes of 5 um or more, or crushing large particles to a size equal to or smaller than the mesh holes by applying pressure, and examples thereof include passing through Filcon S Syringe of a 10 um mesh (BD Medimachine).

The heating treatment is a treatment of heating at 25 to 50°C, and examples thereof include leaving the microorganism to stand in a constant temperature water bath at 40°C for 10 minutes.

The homogenizer treatment is a machine stirring treatment using a homogenizer, and for example, stirring is performed with a T10 basic ULTRA-TURRAX homogenizer (IKA) equipped with a S10N-10G generator (IKA) at 30,000 rpm for 1 minute.

The stomacher treatment is a machine stirring treatment using a stomacher, and for example, the microorganisms are put in a plastic bag and treated with a stomacher Pro-media SH-IIM (ELMEX LIMITED) for 10 minutes.

The ultrasonic treatment is preferably performed using an ultrasonic treatment apparatus for 10 to 30 minutes at a frequency of 20 to 200 kHz, and examples thereof include a treatment in an ultrasonic bath US-104N (oscillation: 38 kHz, output: 150 W) for 10 minutes.

The centrifugation in the centrifugation step of the step (b) is not limited as long as it is performed under conditions that cells of a microorganism precipitate, and the centrifugation is performed at 4°C to 40°C, preferably at 25°C, with a centrifugal force of 1,000 × g or more, preferably 5,000 to 10,000 × g at maximum rotation radius (Rmax), for 5 to 30 minutes, preferably for about 15 minutes.

As shown in reference examples described later, 90% or more of the proteins in the specimen can be removed by performing the steps (a) and (b).

After completion of the centrifugation in the step (b), the supernatant is removed, and the remaining precipitate is subjected to a washing and collecting operation (step (c)) .

The washing operation can be performed by adding a washing liquid in an amount about 10 to 500 folds the amount of the solution containing the precipitate, and then suspending the precipitate by pipetting and/or with a vortex mixer, centrifuging the suspension, and collecting the resultant.

Here, as the washing liquid to be used, it is preferable to use the same buffer solution as that used in the step (a); however, for example, water, peptone physiological saline, peptone water, PBS (phosphate-buffered saline), a Ringer's solution, physiological saline, and a Mitsuoka's buffer can also be used.

In addition, conditions of the centrifugation performed in this step are preferably the same as those of the centrifugation in the step (b), and examples thereof include conditions of 5,000 × g for about 15 minutes at room temperature. In the washing and collecting operation, the addition of the washing liquid and the centrifugation can be performed multiple times until removal of milk proteins is sufficiently achieved.

Thus, according to the method of the present invention, the microorganisms can be efficiently collected from the specimen containing the fermented milk. Accordingly, by using this method, it is possible to more quickly and reliably perform, for example, a test or a microscopic observation in which a milk component has conventionally made measurement or observation of fermented dairy product difficult; evaluation of various activities of the microorganisms in the fermented milk in cases where the influence of the milk component is reduced; verification of the benefit of the fermented dairy product by measuring a viable cell count or measuring a total cell count including viable cells and dead cells; determination of whether the specimen satisfies a set standard from the viable cell count and the total cell count, or determination of presence or absence of a lot difference of the specimen; determination of presence or absence of contaminating microorganisms in the specimen, or measurement of the number thereof; and verification of a storage state and deterioration of the specimen from the ratio between the viable cells and the dead cells.

Hereinafter, contents of the present invention will be further described in detail with reference to examples; however, the present invention is not limited thereto at all.

### Examples

### Reference Example 1 Removal of proteins in acidified milk (1)

(1) Reconstituted skim milk (100 (w/v) skim milk) prepared by dissolving skim milk powder in water was heated and sterilized at 121°C for 15 minutes. A final concentration of 0.8% (w/v) of DL-lactic acid (FUJIFILM Wako Pure Chemical Corporation) was added to the sterilized skim milk to obtain a simulated fermented milk (hereinafter, "acidified milk") adjusted to a pH of 4.4. In addition, an amount of proteins in 1.0 mL of the acidified milk was quantified by TaKaRa BCA Protein Assay Kit (Takara Bio Inc.).
(2) The potassium phosphate buffer solution (pH 7.0) of 50 mM was added to 1.0 mL of the acidified milk in (1), and a mixture was diluted such that the protein concentrations were 11.9 mg/mL, 5.9 mg/mL, 3.0 mg/mL, 1.5 mg/mL, and 0.7 mg/mL. The samples were then centrifuged at 5,000 × g for 15 minutes at room temperature. The supernatant was discarded, and 1 mL of pure water was added, and the amount of proteins in a suspension was measured. Note that the amount of proteins was quantified by TaKaRa BCA Protein Assay Kit (Takara Bio Inc.), and defined as the residual amount of proteins. In addition, the value obtained by the calculation formula "(1 - residual amount of proteins / amount of proteins in 1.0 mL of acidified milk in (1)) × 100" was taken as the removal rate.

### (3) Results

As shown in Table 1, when the protein concentration after the dilution was 3.0 mg/mL or less, about 92% or more of the proteins were removed, and particularly when the protein concentration was 1.5 mg/mL or less, about 98% or more of the proteins were removed, and the residual amount of the proteins was greatly reduced.

**[Table 1]**

| Milk concentration after dilution (%) | Protein concentration after dilution (mg/mL) | Residual amount of proteins (µg) | Removal rate (%) |
|---|---|---|---|
| 4.0 | 11.9 | 16557 | 44.3 |
| 2.0 | 5.9 | 8379 | 71.8 |
| 1.0 | 3.0 | 2383 | 92.0 |
| 0.5 | 1.5 | 615 | 97.9 |
| 0.25 | 0.7 | 607 | 98.0 |

### Reference Example 2 Removal of proteins in acidified milk (2)

In the same manner as in Reference Example 1 (2), the potassium phosphate buffer solution (pH 7.0) of 25 mM to 100 mM, the Tris-HCl buffer solution (pH 7.0) of 100 mM to 1000 mM, and the HEPES buffer solution (pH 7.0) of 50 mM to 500 mM were each added to 1.0 mL of the acidified milk, and the mixture was diluted such that the protein concentration was 3.0 mg/mL. The sample was then centrifuged at 5,000 × g for 15 minutes at room temperature. The supernatant was discarded, and 10 mL of the same buffer solution was added, and the precipitate was suspended by pipetting and with a vortex mixer. Again, the mixture was centrifuged at 5,000 × g for 15 minutes at room temperature, and the supernatant was discarded, and the precipitate was resuspended, and then the mixture was centrifuged under the same conditions. Subsequently, the supernatant was discarded, and 1 mL of pure water was added, and the amount of the proteins in the suspension was measured and taken as the residual amount of proteins. In addition, the removal rate was determined in the same manner as in Reference Example 1. Results are shown in Table 2.

As shown in Table 2, the proteins were removed even when the type and concentration of the buffer solution were changed. In addition, the protein removal rate was increased compared with Reference Example 1 by adding the washing step after the first dilution step and the first centrifugation step.

**[Table 2]**

| Buffer solution (concentration) | Residual amount of proteins (mg) | Removal rate (%) |
|---|---|---|
| Potassium phosphate buffer solution (25 mM) | 1.0 | 97.3 |
| Potassium phosphate buffer solution (50 mM) | 0.9 | 97.4 |
| Potassium phosphate buffer solution (100 mM) | 1.6 | 95.6 |
| Tris-HCl buffer solution (100 mM) | 0.2 | 99.5 |
| Tris-HCl buffer solution (250 mM) | 0.1 | 99.6 |
| Tris-HCl buffer solution (500 mM) | 0.2 | 99.5 |
| Tris-HCl buffer solution (1000 mM) | 0.1 | 99.7 |
| HEPES buffer solution (50 mM) | 0.3 | 99.1 |
| HEPES buffer solution (100 mM) | 0.1 | 99.6 |
| HEPES buffer solution (250 mM) | 0.6 | 98.3 |
| HEPES buffer solution (500 mM) | 1.4 | 96.0 |

### Reference Example 3 Removal of proteins in acidified milk (3)

In the same manner as in Reference Example 1 (2), the potassium phosphate buffer solution (pH 7.0) of 50 mM was added to 1.0 mL of the acidified milk, and the mixture was diluted such that the protein concentration was 3.0 mg/mL. The diluted sample was subjected to treatments 1) to 5) below, respectively.
1) Mesh treatment: passing through Filcon S Syringe of a 10 um mesh (BD Medimachine) 30 times.
2) Heating treatment: leaving the sample to stand in a constant temperature water bath at 40°C for 10 minutes.
3) Homogenizer treatment: stirring with a T10 basic ULTRA-TURRAX homogenizer (IKA) equipped with a S10N-10G generator (IKA) at 30,000 rpm for 1 minute.
4) Stomacher treatment: putting the sample in a plastic bag, and treating with a stomacher Pro-media SH-IIM (ELMEX LIMITED) for 10 minutes.
5) Ultrasonic treatment: treating in an ultrasonic bath US-104N (oscillation: 38 kHz, output: 150 W) for 10 minutes.

The treated sample was then centrifuged at 5,000 × g for 15 minutes at room temperature. The supernatant was discarded, and 1mL of pure water was added, and the amount of proteins in the suspension was measured and taken as the residual amount of the proteins. In addition, the removal rate was determined in the same manner as in Reference Example 1. Results are shown in Table 3.

As can be seen from Table 3, the protein removal rate was increased in any treatment, and the residual amount was reduced by half compared with a case where no treatment was performed.

**[Table 3]**

| Treatment conditions | Residual amount of proteins | (µg) Removal rate (%) |
|---|---|---|
| No Treatment | 2383 | 92.0 |
| Mesh | 790 | 97.3 |
| Heating | 1095 | 96.3 |
| Homogenizer | 850 | 97.1 |
| Stomacher | 622 | 97.9 |
| Ultrasonic | 1064 | 96.4 |

### Example 1 Collection of cells from fermented milk

### (1) Preparation of fermented milk

Fermented milk (pH 4.6) was obtained by inoculating a final concentration of 0.1% (v/v) of LcS (Lactobacillus casei YIT 9029) into skim milk of 10% (w/v) sterilized by heating at 121°C for 15 minutes, and culturing the mixture at 37°C for 48 hours under aerobic conditions, and then defined as "LcS fermented milk". Similarly, fermented milk (pH 4.3 and pH 4.0 respectively) was obtained by inoculating a final concentration of 0.1% (v/v) of LL (Lactococcus lactis YIT 2027) or ST (Streptococcus thermophilus YIT 2001) each and culturing mixtures at 30°C (LL) or 37°C (ST) for 24 hours under aerobic conditions, and then defined as "LL fermented milk" and "ST fermented milk" respectively.

In addition, fermented milk (pH 4.7) was obtained by inoculating a final concentration of 1.0% (v/v) of BB (Bifidobacterium breve YIT 12272) into a milk medium for Bifidobacteria (skim milk powder of 12% (w/v), calcium carbonate of 0.2%, yeast extract of 0.1%, cysteine hydrochloride of 0.03%) sterilized by heating at 115°C for 20 minutes under anaerobic conditions and culturing the mixture at 37°C for 11 hours under anaerobic conditions, and then defined as "BB fermented milk".

The amount of proteins, the total cell count, and the viable cell count in 1.0 mL of each of the fermented milk were measured, and the measured values were defined as the amount of proteins before the dilution, the total cell count before the dilution, and the viable cell count before the dilution. The total cell count and the viable cell count were measured as follows.

(2) A potassium phosphate buffer solution (pH 7.0) of 50 mM was added to 1.0 mL of the fermented milk in (1), and the mixture was diluted such that the protein concentration was 0.7 mg/mL. The sample was then centrifuged at 5,000 × g for 15 minutes at room temperature. The supernatant was discarded, 10 mL of the same buffer solution was added, and the precipitate was suspended by pipetting and with a vortex mixer. Then, the mixture was centrifuged at room temperature for 15 minutes at 5,000 × g, and the supernatant was discarded. 1 mL of pure water was added, the amount of proteins, the total cell count, and the viable cell count in the suspension were measured, and the measured values were defined as the residual amount of the proteins, the residual total cell count, and the residual viable cell count. In addition, the value obtained by the calculation formula "1 - (residual amount of proteins / amount of proteins before dilution) × 100" was defined as the protein removal rate, and the value obtained by the calculation formula "residual total cell (viable cell) count / total cell (viable cell) count before dilution × 100" was defined as the total cell (viable cell) count collection rate.

(3) In (2), the fermented milk was diluted such that the protein concentration was 0.7 mg/mL, and then subjected to the mesh treatment (passing through the Filcon S Syringe of a 10 um mesh 30 times), and subsequent operations were performed in the same manner as in (2), and the residual amount of the proteins, the residual total cell count, and the residual viable cell count were measured.

### <Measurement of total cell count>

Counts were made by nucleic acid staining method with DAPI. 5 µL of the sample diluted 20 to 50 folds with 70% ethanol was applied to each well of MAS coated slide glass (Matsunami Glass Ind., Ltd.) having 1 cm × 1 cm wells. After air-drying, 4.5 µL/well of VECTASHIELD Mounting Medium for Fluorescence with DAPI H-1200 (Vector Laboratories, Inc.) was applied, and a cover glass (Matsunami Glass Ind., Ltd.) was put thereon from above. An optical microscope Leica DM5500 B (Leica Biosystems) was used for observing the slide glass, and a Leica MMAF system (Leica Biosystems) was used for acquisition of fluorescence images. Fluorescence of DAPI (excitation light: 358 nm, emission fluorescence: 461 nm) from 10 visual fields per well was acquired in monochrome using an A4 fluorescence filter (for DAPI), and then subjected to an image analysis using an image analysis software Image-Pro Plus ver. 6.1 (Nippon Roper K.K.), and the number of cells per 1 mL of the diluted solution was calculated. An average value of 4 to 8 squares was multiplied by the dilution factor to obtain the number of cells (cells/mL) in a sample.

### <Measurement of viable cell count>

The sample appropriately diluted with an aqueous solution of 0.85% (w/v) sodium chloride (physiological saline) was applied to an MRS plate medium (BA-30 agar (Ina Food Industry Co., Ltd.)1.5% (w/v) was added to BD Difco Lactobacillus MRS broth) using a spiral plater EDDY JET 2 (IUL Instruments GmbH).

The plate medium spread with the bacteria was cultured at 37°C for 2 days or more under aerobic conditions. The number of colonies grown was measured with an automatic counting device ProtoCOL 3 (Synoptics Ltd.), and the number of colony forming units (CFU) per 1 mL of cell solution was calculated from the dilution factor and taken as the viable cell count. Note that since the operation was performed under aerobic conditions and the aerobic condition was expected to have a large influence on the viable count of BB, the viable cell count for BB was not measured.

### (4) Results

As shown in Table 4, most of the proteins were removed from the fermented milk of any genus of bacteria, and more than half of the cells were collected. In addition, it was considered that the total cell count collection rate of BB exceeded 100% because clumps of bacteria which strongly adhered to curds might have dispersed along with the solubilization of the milk proteins, and thus the apparent collected cell count was increased. In addition, in cases except for BB, there was a tendency that the collection rate of cells was increased by performing the mesh treatment.

**[Table 4]**

| Strain | Mesh treatment | Removal rate of proteins % | Total cell count collection rate (%) | Viable cell count collection rate (%) |
|---|---|---|---|---|
| LcS fermented milk | NO | 98.7 | 53.8 | 73.9 |
| | YES | 98.2 | 72.3 | 84.0 |
| LL fermented milk | NO | 98.1 | 59.0 | 66.7 |
| | YES | 98.3 | 61.5 | 82.4 |
| ST fermented milk | NO | 92.0 | 54.2 | 57.5 |
| | YES | 94.2 | 64.3 | 81.3 |
| BB fermented milk | NO | 96.0 | 154.1 | - |
| | YES | 96.5 | 146.9 | - |

### Example 2 Collection of cells from dairy products

(1) 50 mM potassium phosphate buffer solution (pH 7.0) was added to 50 mL of "Yakult 400LT" (Yakult Honsha Co., Ltd.) (hereinafter, also referred to as the product), which is a product using fermented milk containing LcS as an ingredient, and the mixture was diluted such that the protein concentration was about 1.6 mg/mL. After passing the sample through the mesh (Filcon S Syringe 10 µm) twice, the sample was centrifuged at 5,000 × g for 15 minutes at room temperature. The supernatant was discarded, and the same buffer solution was added at a ratio of 2 mL to the precipitate deriving from 1 mL of the sample, and the precipitate was suspended by pipetting and with a vortex mixer. Then, the mixture was centrifuged at room temperature for 15 minutes at 5,000 × g, and the supernatant was discarded. Subsequently, 1 mL of pure water was added, and the residual total cell count and the residual viable cell count in the suspension (collection solution) were measured in the same manner as in Example 1. In addition, the total cell count before the dilution and the viable cell count before the dilution per 1.0 mL of the product were measured, and the collection rate was determined by the calculation formula "residual total cell (viable cell) count / total cell (viable cell) count before dilution × 100".
(2) Results

No obvious milk protein residue was observed judging from the appearance, and as shown in Table 5, the collection rate was 80 to 94% for both the total cell count and the viable cell count, and the LcS could be collected from dairy products without a large loss in the number of bacteria or damage to the cells.

**[Table 5]**

| Product lot | Sample | Nucleic acid staining method | | Colony counting method | |
|---|---|---|---|---|---|
| | | Total cell count (cells) | Collection rate (%) | Viable cell count (CFU) | Collection rate (%) |
| NFJB | Product | 4.5 × 10¹⁰ | 83 | 4.4 × 10¹⁰ | 80 |
| | Collection collection solution | 3.8 × 10¹⁰ | | 3.5 × 10¹⁰ | |
| VDHA | Product | 4.7 × 10¹⁰ | 90 | 5.0 × 10¹⁰ | 94 |
| | Collection solution | 4.2 × 10¹⁰ | | 4.7 × 10¹⁰ | |
| NABB | Product | 5.7 × 10¹⁰ | 86 | 5.9 × 10¹⁰ | 87 |
| | Collection solution | 4.9 × 10¹⁰ | | 5.2 × 10¹⁰ | |

## Claims

1. A method for collecting microorganisms from a specimen containing fermented milk, the method comprising, in this order, steps of: (a) diluting the specimen with a buffer solution such that a protein concentration is 3 mg/mL or less; (b) performing centrifugation; and (c) washing and collecting the microorganisms.

2. The method according to claim 1, wherein the specimen is diluted such that the protein concentration is 2 mg/mL or less.

3. The method according to claim 1 or 2, wherein the buffer solution has a pH of 6.5 to 7.5.

4. The method according to any one of claims 1 to 3, wherein the buffer solution is a potassium phosphate buffer solution, a Tris-HCl buffer solution, or a HEPES buffer solution.

5. The method according to any one of claims 1 to 4, wherein the concentration of the buffer solution is 25 mM to 1000 mM.

6. The method according to claim 4, wherein the buffer solution is a potassium phosphate buffer solution having a concentration of 25 mM to 100 mM.

7. The method according to any one of claims 1 to 6, wherein the step (a) is followed by a treatment selected from a mesh treatment, a heating treatment, a homogenizer treatment, a stomacher treatment, and an ultrasonic treatment.

8. The method according to any one of claims 1 to 7, wherein the microorganisms are viable.

9. The method according to any one of claims 1 to 8, wherein the specimen is an acidic milk beverage.
